# EUROPÄISCHE PATENTSCHRIFT

(11) **EP 1 410 039 B1**
(45) Veröffentlichungstag und Bekanntmachung des Hinweises auf die Patenterteilung: **22.12.2010**
(21) Anmeldenummer: 02762152.3
(22) Anmeldetag: 23.07.2002
(51) Int. Cl.: G01N 33/68

(54) **VERFAHREN ZUR DIAGNOSE VON NIERENSCHÄDIGUNGEN IM FRÜHSTADIUM**
METHOD FOR THE DIAGNOSIS OF KIDNEY DAMAGE IN THE EARLY STAGES
PROCEDE DE DIAGNOSTIC DE LESIONS RENALES AU STADE PRECOCE

(30) Priorität: 26.07.2001 AT 11672001
(43) Veröffentlichungstag der Anmeldung: 21.04.2004
(73) Patentinhaber: Vitateq Biotechnology GmbH, 6020 Innsbruck (AT)
(72) Erfinder: KRONENBERG, Florian, A-6020 Innsbruck (AT)
(74) Vertreter: Sonn & Partner Patentanwälte
(86) Internationale Anmeldenummer: PCT/AT2002/000219
(87) Internationale Veröffentlichungsnummer: WO 2003/010544

(56) Entgegenhaltungen:
- DIEPLINGER H ET AL: "PLASMA APOLIPOPROTEIN A-IV METABOLISM IN PATIENTS WITH CHRONIC RENAL DISEASE" EUROPEAN JOURNAL OF CLINICAL INVESTIGATION, Bd. 22, Nr. 3, 1992, Seiten 166-174, XP002236440 ISSN: 0014-2972
- SEISHIMA M ET AL: "AN INCREASED APO-A-IV SERUM CONCENTRATION OF PATIENTS WITH CHRONIC RENAL FAILURE ON HEMODIALYSIS" CLINICA CHIMICA ACTA, Bd. 167, Nr. 3, 1987, Seiten 303-311, XP002236441 ISSN: 0009-8981
- KRONENBERG FLORIAN ET AL: "Low apolipoprotein A-IV plasma concentrations in men with coronary artery disease." JOURNAL OF THE AMERICAN COLLEGE OF CARDIOLOGY, Bd. 36, Nr. 3, September 2000 (2000-09), Seiten 751-757, XP001146847 ISSN: 0735-1097

## Beschreibung

Die Erfindung bezieht sich auf ein Verfahren zur Diagnose von Nierenschäden im frühen Stadium.

Das Glykoprotein Apolipoprotein A-IV (ApoA-IV) wird fast ausschließlich in den Enterozyten im menschlichen Darm gebildet und in die Lymphe sekretiert (1). Es ist ein strukturelles Protein von Chylomikronen, und die mittleren Plasmawerte liegen bei etwa 15 mg/dl (2). In nüchternem Zustand zirkuliert der Großteil des ApoA-IV im Plasma als Teil eines lipidarmen, kleinen, HDL-ähnlichen Partikels, das kein ApoA-I enthält (3, 4). Die physiologische Funktion von ApoA-IV ist unklar bzw. wird kontroversiell diskutiert. Es ist postuliert worden, dass es an der Fettabsorption (5) und der Regulierung der Nahrungsaufnahme (6) beteiligt ist, keiner dieser Befunde konnte jedoch in genetisch modifizierten Mäusen bestätigt werden (7, 8). In vitro-Studien berichteten von überzeugenden Beweisen dafür, dass ApoA-IV an mehreren Schritten des reversen Cholesterintransportweges beteiligt ist, der Cholesterin aus peripheren Zellen entfernt und zur Metabolisierung zur Leber und den steroidogenen Organen leitet (9 - 11). ApoA-IV aktiviert die Lezithin-Cholesterinacyltransferase (LCAT) (12, 13) und moduliert die Aktivierung von Lipoproteinlipase (14) sowie den von CETP vermittelten Transfer von Cholesterylestern von HDL zu LDL (15), was darauf hinweist, dass ApoA-IV einen anti-atherogenen Faktor darstellen könnte. Dies wird von Studien an Tieren sowie an Menschen unterstützt. Mit Fett gefütterte Mäuse, die ApoA-IV entweder von Menschen (16) oder von Mäusen (17) überexprimieren, zeigten im Vergleich zu Kontrollmäusen eine signifikante Reduktion atherosklerotischer Läsionen der Aorta. Dies wurde sogar dann beobachtet, wenn ApoA-IV in ApoE-defizienten Mäusen mit hochgradig atherogenem Hintergrund überexprimiert wurde (16).

Einige Studien untersuchten ApoA-IV bei Nierenpatienten und beobachteten einen Anstieg von ApoA-IV. Nestel et al. (18) sowie Seishima und Muto (19) beschrieben deutlich erhöhte ApoA-IV-Konzentrationen in kleinen Gruppen von Hämodialyse- und CAPD-Patienten. Dies wurde von Dieplinger et al. bestätigt, die signifikante Unterschiede in der Plasmaverteilung von ApoA-IV mit einer Ansammlung von ApoA-IV in den hochdichten Lipoproteinen (HDL) im Vergleich zu Kontrollen (20) beobachteten. Sie vertraten die Meinung, dass diese veränderte Verteilung den gestörten reversen Cholesterintransport bei diesen Patienten widerspiegeln könnte (21). In einer großen Studie an mehreren Zentren an 534 Hämodialyse- und 168 CAPD-Patienten wurden im Vergleich zu Kontrollen etwa zweifach erhöhte ApoA-IV-Konzentrationen beobachtet (22). Sobald das Endstadium der Nierenerkrankung erreicht ist, scheint es keine Unterschiede in den ApoA-IV-Konzentrationen zwischen den beiden Dialysebehandlungsmodalitäten sowie zwischen diabetischen und nicht-diabetischen Patienten zu geben (22). Es ist jedoch nicht bekannt, in welchem Stadium der Nierenschädigung ApoA-IV anzusteigen beginnt, und ob es auch in dieser Erkrankungspopulation mit atherosklerotischen Komplikationen in Zusammenhang steht. Seishima et al. (Clin. Chim. Acta 167 (1987), 303-311) untersuchten ApoA-IV bei Patienten mit terminaler Niereninsuffizienz, die mit Hämodialyse behandelt werden, also bei einer Erkrankung der Niere im Endstadium (Nierenersatztherapie).

In Seishima et al. (J. Lipid Res. 33 (1992), 1441-1447) wurden 7 Ratten mit 5/6 Nierenentfernung mit 7 Kontrollratten verglichen. Durch diesen Eingriff wurde eine experimentelle Nierenfunktionseinschränkung erzeugt, was sich in einer deutlichen Erhöhung des BUN (15,2 vs. 56,1 mg/dl) und Serum-Kreatinin (0,69 vs. 1,46 mg/dl) zeigte. ApoA-IV war doppelt so hoch bei den 5/6-nephrektomierten Ratten, wobei keine absoluten Konzentrationen, sondern nur eine arbiträre Einheit angegeben wurden.

Es ist aber bekannt, dass die Ratte ein klassisches HDL-Tier ist (und ApoA-IV hauptsächlich HDL-assoziiert ist) und sich daher Ergebnisse nicht auf den Menschen übertragen lassen, bei denen hauptsächlich LDL-haltige Lipoproteine vorgefunden werden.

In Massy et al. (Clin. Nephrol. 55 (2001), 156-158) wurden die ApoA-IV-Konzentrationen in 36 nierentransplantierten Patienten und 20 Kontrollpersonen untersucht. Die Kreatinin-Clearance wurde errechnet und nicht direkt gemessen. Zudem standen die Patienten unter dem Einfluss von drei potenten Immunsuppressiva (Cyclosporin, Azathioprin und Kortikosteroide), die alle einen deutlichen und belegten Einfluss auf den Lipoproteinstoffwechsel beim Menschen haben. Patienten, die Cyclosporin erhielten, hatten zudem höhere ApoA-IV-Werte als jene ohne Cyclosporin. Ob dieser Effekt durch Cyclosporin selbst oder durch einen Einfluss von Cyclosporin auf die Nierenfunktion hervorgerufen wurde, wurde nicht gezeigt.

Es zeigte sich, dass diese beiden, im Stand der Technik offenbarten Modelle (jenes der Nierentransplantation und jenes der 5/6-nephrektomierten Ratten) ungeeignet sind, um einen ApoA-IV als frühen Parameter einer eingeschränkten Nierenfunktion zu zeigen. Für die terminale Nierenfunktion kann dies per definitionem ohnehin nicht erwartet werden.

Frühe Diagnosen sind immer wichtig, insbesondere bei Nierenerkrankungen. Ein Ziel der vorliegenden Erfindung besteht daher darin, ein Verfahren zur Diagnose von Nierenschädigungen im Frühstadium zu entwickeln, d.h. zu einem Zeitpunkt im Verlauf der Nierenerkrankung, wo die Nierenschädigung noch geringfügig ist und der Beginn einer Therapie größere Erfolge verspricht.

Dieses Ziel wird durch ein Verfahren zur Diagnose von Nierenschäden im Frühstadium beim Menschen erreicht, das durch die folgenden Schritte gekennzeichnet ist:
- Messen der Menge von ApoA-IV in einer Körperflüssigkeits- oder Gewebeprobe eines Menschen und
- Vergleichen der gemessenen Menge von ApoA-IV mit einem Referenzwert.

Erstaunlicherweise konnte im Zusammenhang mit der vorliegenden Erfindung gezeigt werden, dass der Anstieg der ApoA-IV-Konzentration in den sehr frühen Stadien von verschiedenen Nierenerkrankungen auftritt. Wie bei vielen anderen Krankheiten weisen Patienten in einem späten Stadium eine Vielzahl unphysiologischer Werte von Proteinen auf (unter anderem ApoA-IV), es ist jedoch von keinem davon erwiesen, dass er ein signifikanter Marker in den frühen Stadien einer Nierenschädigung ist. Tatsächlich ist auch der Verdacht geäußert worden, dass erhöhte ApoA-IV-Konzentrationen bei Patienten mit Nierenerkrankungen im Endstadium, die eine Dialyse erhalten, mit diabetischen Komplikationen und Prozessen, die an der Dialysebehandlung beteiligt sind, in Zusammenhang stehen.

Es ist sehr interessant, dass ApoA-IV in den frühen Stadien einer Nierenschädigung erhöht ist, während andere Marker des (Fett-)Metabolismus, wie HDL-Cholesterin, ApoA-I oder ApoB, für solch ein frühes Stadium keine signifikante Markerfunktion haben. Tatsächlich waren die HDL- und LDL-Cholesterinwerte unverändert, während die ApoA-I- und ApoB-Konzentrationen bei Patienten vermindert waren. Die signifikanteste Veränderung bei frühen Nierenerkrankungen war jedoch ein Anstieg der ApoA-IV-Konzentrationen, die bei Patienten fast 70% höher lag als bei Kontrollen.

Daher ist bereits eine geringfügige Schädigung durch eine Nierenerkrankung von einem signifikanten Anstieg der ApoA-IV-Konzentrationen begleitet, was ApoA-IV zu einem relevanten und signifikanten Marker für eine Nierenschädigung im frühen Stadium macht. Nierenschäden im Frühstadium können z.B. von einem GFR-Bereich von über 90 ml/min/1,73 m² definiert werden. Ein mittlerer GFR von 120 ml kann ebenfalls als Situation eines Nierenschadens im frühen Stadium betrachtet werden.

Im Rahmen der vorliegenden Erfindung wurden Patienten von den frühesten bis zu den fortgeschrittenen Stadien der Nierenfunktionseinschränkung untersucht. Diese Funktionseinschränkung lässt sich zum Teil mit herkömmlichen Messmethoden gar nicht feststellen (z.B. ist in diesen Phasen das Serumkreatinin oft noch normal, oftmals ist sogar die glomeruläre Filtrationsrate noch normal). Dies alleine zeugt bereits von der Einzigartigkeit der Studie. Die Nierenfunktion wurde mit einem "gold standard" festgestellt (Iohexol-Clearance). Der Großteil der Literatur, der andere Parameter in diesen Phasen der Niereninsuffizienz gemessen hat, verwendete hauptsächlich rechnerische Methoden zur Feststellung der Nierenfunktion, in die Parameter wie Kreatinin und Alter eingehen. Es ist aber hinreichend bekannt, dass Kreatinin erst zu steigen beginnt, wenn die Nierenfunktion um mehr als 50 % eingeschränkt ist. Dieser Kreatinin-blinde Bereich wird im Rahmen der vorliegenden Erfindung allerdings durch ein Clearance-Verfahren voll erkannt. Zudem wird gemäß der erfindungsgemäß durchgeführten Untersuchungen die Diagnose der Erkrankung in den meisten der Patienten durch Biopsie oder Ultraschall klar gesichert. Erstmals wurde erfindungsgemäß damit eine Studie zur Verfügung gestellt, in der Nierenfunktion und Nierengrunderkrankung mit akzeptiert validen Methoden festgestellt wurde.

Der Referenzwert, mit dem die Menge ApoA-IV in einer Probe verglichen wird, wird vorzugsweise von einem Menschen erhalten, der keine Nierenschäden hat (gesunder Mensch). Eine Nierenschädigung kann dann an Hand eines erhöhten Wertes von ApoA-IV in der Körperflüssigkeits- oder Gewebeprobe des zu untersuchenden Menschen diagnostiziert werden, z.B. eine Person, von der angenommen wird, dass sie einen Nierenschaden hat.

Das erfindungsgemäße Verfahren ist besonders für die routinemäßige Analyse von gesunden Menschen geeignet, d.h. Analysen, die jährlich durchgeführt werden, um Nierenschäden in einem sehr frühen Stadium zu erkennen, d.h. zu einem Zeitpunkt, wenn die Wahrscheinlichkeit, Menschen durch therapeutische Maßnahmen zu heilen, üblicher Weise sehr hoch ist.

Ein erhöhter ApoA-IV-Wert wird als ein Wert angesehen, der höher ist als in einer gesunden Kontrollperson. Vorzugsweise ist dieser erhöhte Wert mindestens 20%, vorzugsweise mindestens 50%, insbesondere mindestens 70% höher als der Referenzwert, der von einem Menschen ohne Nierenschäden genommen wird. Der optimale "gesunde" Referenzwert ist ein Referenzwert, der vom selben Menschen in einem gesunden Stadium erhalten wurde, d.h. zu einem früheren Zeitpunkt, wo der Mensch noch keine Nierenschäden hatte.. Überdies hängt der relative Nachweis erhöhter Werte auch vom Verfahren ab, mit dem die Menge ApoA-IV gemessen wird, d.h. der Empfindlichkeit des relativen Tests, der Reproduzierbarkeit etc., einem wichtigen Faktor zur Definition des Diagnoseergebnisses.

Der Vergleich des gemessenen Wertes beim Menschen, der mit der vorliegenden Erfindung diagnostiziert werden soll, und des Referenzwertes ist auch von der Körperflüssigkeit oder dem Gewebe abhängig, von der/dem die Werte genommen und gemessen werden. Vorzugsweise wird natürlich Serum getestet, worin der (gesunde) Referenzwert üblicher Weise zwischen 10 und 18 mg ApoA-IV/dl Serum beträgt, wenn mit ApoA-IV ELISA getestet wird. In einem solchen System kann ein frühes Stadium einer Nierenschädigung an Hand eines Wertes von höher als 18, vorzugsweise höher als 22, insbesondere höher als 28 mg/ApoA-IV/dl Serum festgestellt werden. Wie oben ausgeführt ist, sind diese absoluten Werte jedoch immer von der sehr spezifischen Art der Messung der Menge von ApoA-IV und der Körperflüssigkeit bzw. dem Gewebe, worin die Menge ApoA-IV gemessen wird, abhängig.

ApoA-IV-Konzentrationen die im Serum gemessen werden, weisen eine extrem hohe Korrelation mit jenen Werten, die im Plasma gemessen werden, auf. Die Unterschiede von einigen wenigen Prozenten lassen sich durch die übliche Beobachtung erklären, dass Werte im Serum gemessen generell um wenige Prozente höher sind als jene, die im Plasma gemessen werden. Es ist also vollkommen gleich, ob man die Messungen in Plasma oder Serum durchführt, solange man die Ergebnisse mit einem Referenzkollektiv vergleicht, in welchem die Messungen im gleichen "Medium" durchgeführt wurden.

Andere bevorzugte Quellen von Material, das erfindungsgemäß getestet werden soll, als von Blut abgeleitete Körperflüssigkeiten, können Nierengewebe, insbesondere Biopsiematerial von Nierengewebe, sowie Lymphe, einschschließen.

Mit dem erfindungsgemäßen Verfahren kann ein frühes Stadium jeder Nierenschädigung diagnostiziert werden, unabhängig von der spezifischen Pathologie einer gegebenen Nierenerkrankung. Vorzugsweise handelt es sich bei diesem Frühstadium einer Nierenschädigung um eine Glomerulonephritis im Frühstadium, eine polyzystische Nierenerkrankung im Frühstadium, eine chronische "Pyelonephritis" im Frühstadium oder eine diabetische Nephropathie im Frühstadium.

Es ist weiters mit der vorliegenden Erfindung analysiert worden, dass Menschen mit Erkrankungen der Koronararterien oder Menschen mit atherosklerotischen Komplikationen im frühen Stadium einer Nierenschädigung im Vergleich zu gesunden Menschen keine so signifikant erhöhte ApoA-IV-Konzentration haben. Dies ist dadurch bedingt, dass bei einem Menschen, das eine solche Erkrankung der Koronararterien oder atherosklerotische Komplikationen hat, ApoA-IV vermindert ist, wenn keine Nierenschädigung vorliegt. Daher muss die Diagnose einer Nierenschädigung im Frühstadium bei solchen Menschen ihren Voraussetzungen entsprechend angepasst werden und sollte unter Berücksichtigung der Referenzwerte von Menschen, die Erkrankungen der Koronararterien oder atherosklerotische Komplikationen, jedoch keine Nierenschäden haben, diagnostiziert werden (solche Referenzwerte für ApoA-IV sind niedriger als bei gesunden Menschen). Wenn daher das vorliegende Verfahren bei Menschen mit Erkrankungen der Koronararterien oder atherosklerotischen Komplikationen durchgeführt wird, sollte vorzugsweise ein Referenzwert, der das Fehlen einer Nierenschädigung anzeigt, vorgesehen werden, der niedriger ist als für Menschen, die keine Nierenschädigungen und keine Erkrankungen der Koronararterie oder atherosklerotische Komplikationen aufweisen. In solchen Fällen ist der niedrigere Referenzwert, der das Fehlen einer Nierenschädigung im Frühstadium anzeigt, mindestens 20%, vorzugsweise mindestens 40%, insbesondere mindestens 60% niedriger als der Referenzwert für Menschen, die keine Nierenschädigung und auch keine Erkrankungen der Koronararterie oder atherosklerotische Komplikationen haben. In diesem Fall kann das Fehlen einer Nierenschädigung im frühen Stadium durch einen geringeren Referenzwert von 6 bis 14 mg ApoA-IV/dl Serum gekennzeichnet sein, wie oben definiert ist. Ein Frühstadium einer Nierenerkrankung kann bei solchen Menschen mit einer Erkrankung der Koronararterie oder mit atherosklerotischen Komplikationen an Hand einen Wertes von höher als 14, vorzugsweise höher als 18, insbesondere höher als 22 mg ApoA-IV/dl Serum diagnostiziert werden.

Gemäß einem anderen Aspekt bezieht sich die vorliegende Erfindung auf die Verwendung eines Verfahrens gemäß einem der Ansprüche 1 bis 11 zur Beobachtung der Entwicklung einer Nierenschädigung im Frühstadium. Daher wird die vorliegende Erfindung auch zur Verwendung von ApoA-IV oder ApoA-IV nachweisenden oder messenden Mitteln zur Diagnose von Nierenschädigungen im frühen Stadium herangezogen.

Gemäß einem weiteren Aspekt bezieht sich die vorliegende Erfindung auch auf die Verwendung eines Kits zur Durchführung des erfindungsgemäßen Verfahrens, wobei dieser Kit umfasst:
- ein Gefäß mit einer Probe von Körperflüssigkeit oder einer Gewebeprobe eines Menschen mit Nierenschädigung oder dem Risiko einer Nierenschädigung, oder ein Gefäß, von dem angenommen wird, dass es mit dieser Probe gefüllt ist,
- Mittel zum Messen der Menge von ApoA-IV in einer Körperflüssigkeits- oder Gewebeprobe und
- ein Referenzwertmittel zur Ermöglichung der Diagnose einer Nierenschädigung im Frühstadium bei diesem Menschen.

Das Mittel zum Messen der Menge von ApoA-IV kann aus jeglichem geeigneten Mittel zum Nachweis/Quantifizieren von ApoA-IV ausgewählt werden, z.B. anti-ApoA-IV-Antikörpern, insbesondere polyklonalen Antikörpern, sekundären Antikörpern, insbesondere enzymatisch oder chemisch markierten sekundären Antikörpern, ApoA-IV-RNA spezifischen Nukleinsäuren, ApoA-IV spezifischen enzymatischen Tests, ApoA-IV spezifischen ELISAs oder Kombinationen davon.

Das Mittel des Referenzwertes, das bei diesem Menschen die Diagnose einer Nierenschädigung im Frühstadium ermöglicht, ist vorzugsweise ausgewählt aus einem Gefäß mit einer vorbestimmten Menge ApoA-IV, einem Gefäß mit einer Körperflüssigkeits- oder Gewebeprobe eines gesunden Menschen, einem Gefäß mit einer Körperflüssigkeits- oder Gewebeprobe eines Menschen mit Nierenschädigung, jeweils vorzugsweise in lyophilisierter Form, einer Kalibrierungskurve, einer Anleitung zur Verwendung des Kits oder Kombinationen davon. Mit dem Kit sollte vorzugsweise auch eine geeignete Anleitung (z.B. in schriftlicher Form) zur Verfügung gestellt werden, worin spezifische Informationen in Bezug auf die Diagnose einer Nierenschädigung im Frühstadium gegeben werden.

Gemäß einem weiteren Aspekt bezieht sich die vorliegende Erfindung auch auf ein Verfahren zur Diagnose von koronaren Herzerkrankungen bei Menschen, die an einer Nierenschädigung im Frühstadium leiden, gekennzeichnet durch die folgenden Schritte:
- Messen der Menge von ApoA-IV in einer Körperflüssigkeits- oder Gewebeprobe eines Menschen und
- Vergleichen der gemessenen Menge ApoA-IV mit einem Referenzwert.

Obwohl gezeigt worden ist, dass ApoA-IV ein relevanter Marker bei der (gesunden) Normalbevölkerung ist, war es erstaunlich, dass sich dieses Protein als verlässlicher Marker für koronare Herzerkrankungen bei Menschen erwies, die an Nierenerkrankungen leiden, da andere Marker für koronare Herzerkrankungen, wie Cholesterin oder HDL-Cholesterin, als CHD-Marker bei Patienten mit Nierenerkrankungen versagten, insbesondere bei Dialysepatienten. Daher ist es ebenfalls möglich, koronare Herzerkrankungen (oder deren Risiko) mit der erfindungsgemäßen Methodik zu analysieren.

Es ist sowohl die Einschätzung von ApoA-IV als Prädiktor für eine Einschränkung der Nierenfunktion als auch als Prädiktor für das Vorliegen einer Atherosklerose oft nur gemeinsam möglich. D.h. falls ein Patient hohes ApoA-IV hat, muss festgestellt werden, ob nicht eine Einschränkung der Nierenfunktion vorliegt. Sind die Werte niedrig, so ist dies ein Hinweis auf ein mögliches Vorliegen von Atherosklerose. Liegt ApoA-IV im "Normalbereich", kann natürlich auch das gleichzeitige Vorhandensein von Nierenfunktionseinschränkung und Atherosklerose vorliegen. Hat z.B. ein Patient mit bekannter Nierenfunktionseinschränkung einen für "Gesunde" relativ normalen ApoA-IV-Wert, so sollte unbedingt noch nach dem Vorhandensein von atherosklerotischen Veränderungen gesucht werden.

Die vorliegende Erfindung wird an Hand der folgenden Beispiele und Zeichnungen weiter charakterisiert, sie ist jedoch nicht darauf beschränkt.

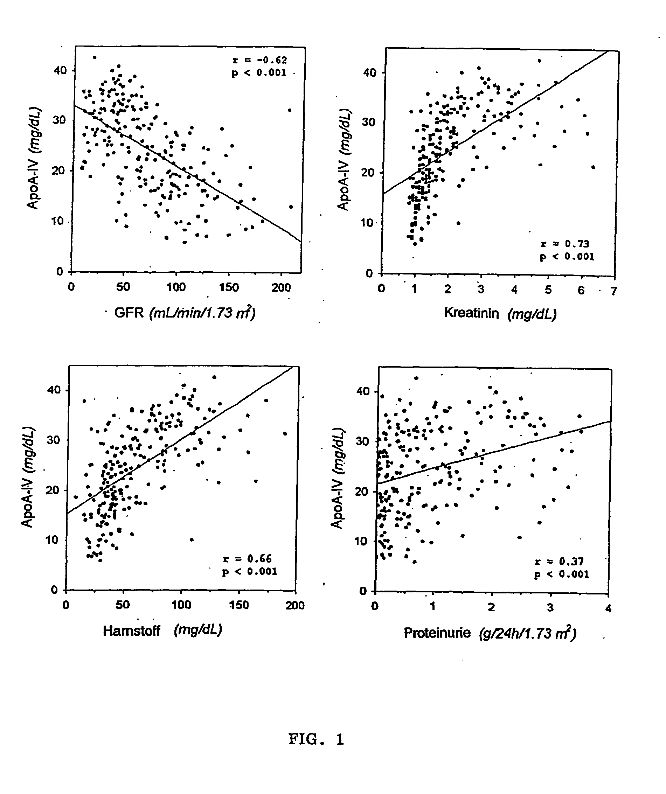

Fig. 1 zeigt die Korrelation von ApoA-IV mit Nierenfunktionsparametern (Serumkreatinin, Serumharnstoff, Glomerulofiltrationsrate (GFR) und tägliche Proteinurie r = 0,37).

### BEISPIELE:

### Verfahren:

### Patienten:

Die Patienten wurden 1997 aus 8 nephrologischen Abteilungen in Deutschland (Göttingen, Greifswald, Heidelberg, Homburg/Saar, München), Österreich (Feldkirch, Innsbruck) und Südtirol (Bozen)
rekrutiert, wobei fast zwei Drittel der Patienten aus zwei Abteilungen (Heidelberg und Innsbruck) kamen (23). Patienten mit weißer Hautfarbe im Alter von 19 bis 65 Jahren, die mindestens ein Mal während des vergangenen Jahres die Ambulanz aufgesucht hatten, wurden in die Studie aufgenommen. Ausschlusskriterien waren Serumkreatinin > 6 mg/dl, Diabetes mellitus, Malignität, Leber-, Schilddrüsen- oder ansteckende Krankheiten zur Zeit der Rekrutierung, nephrotisches Syndrom, definiert als tägliche Proteinurie > 3,5 g/1,73 m², Organtransplantation, Allergie gegen ionische Kontrastmedien und Schwangerschaft. 340 Patienten erfüllten die Kriterien, von welchen 28 nicht erreichbar waren und 85 die Teilnahme an der Studie verweigerten. Die verbleibenden 227 Patienten wurden in die Studie aufgenommen, und ihre Charakteristika sind in Tabelle 1 beschrieben. Die Studie wurde von den institutionellen Ethikkommissionen genehmigt, und die Testpersonen gaben eine schriftliche Einverständniserklärung ab.

**Tabelle 1:**

| **Charakteristika von Patienten mit Nierenerkrankungen und alters- und geschlechtsmäßig angepasste Kontrollen ^{a}** | | |
|---|---|---|
| | **Kontrollen (n = 227)** | **Nierenpatienten (n = 227)** |
| Alter (Jahre) | 45,8 ± 12,3 | 45,7 ± 12,6 |
| Geschlecht, | 73 / 154 | 73 / 154 |
| weiblich/männlich, n | | |
| Körpermasseindex | 26,4 ± 3,6 | 25,2 ± 3, 8 ^{b} |
| GFR, ml/min/1,73 m² | - | 70 ± 42 [38, 63, 96] |
| Kreatinin, mg/dl | 0,99 ± 0,18 | 2,02 ± 1,16 ^{b} |
| Harnstoff, mg/dl | 29 ± 7 | 60 ± 34 ^{b} |
| Proteinurie, g/24 h/1,73 m² | - | 0,9 ± 0,9 [0,2, 0,6, 1,5] |
| Serumalbumin, g/dl | 4,88 ± 0,49 | 4,57 ± 0,41 ^{b} |
| Hämatokrit | - | 0,41 ± 0,06 |
| CRP, mg/dl | - | 0,37 ± 0,76 [0,07, 0,16, 0,41] |
| Systol. Blutdruck, mm Hg | 129 ± 13 | 137 ± 21 ^{b} |
| Diastol. Blutdruck, mm Hg | 81 ± 9 | 87 ± 14 ^{b} |
| medikamentös behandelter Bluthochdruck, % | 10,6 | 78,9 ^{b} |
| Raucher / Exraucher / Nichtraucher, n | 61 / 58 / 108 | 49 / 57 / 121 |

| | | |
|---|---|---|
| ^{a}Daten sind Mittel ± SD und, wo zutreffend, [25. Percentil, Mittel, 75. Percentil]. ^{b} P < 0,001 für den Vergleich mit den Kontrollen. | | |

Um Unterschiede zwischen Beobachtern zu vermeiden, wurden alle Nierenpatienten von einem einzigen Arzt rekrutiert, der die teilnehmenden Zentren besuchte. Die Krankengeschichte der Patienten einschließlich atherosklerotischer Ereignisse wurde in einem Gespräch aufgenommen und mit ihren Unterlagen verglichen. Jeder Patient unterzog sich einer physischen Untersuchung. Die Hauptursachen der Nierenerkrankungen waren eine Glomerulonephritis (GN) bei 97 Patienten (in 90 Fällen durch Biopsie bestätigt), eine polyzystische Nierenerkrankung (polycystic kidney disease, PCKD) bei 37 Patienten, chronische "Pyelonephritis" (PN) bei 24 Patienten, andere Arten von Nierenerkrankungen bei 43 Patienten und unbekannt bei 26 Patienten.

Die Patienten wurden mit 227 alters- und geschlechtsmäßig angepassten Kontrollen mit weißer Hautfarbe und gleicher ethnischer Herkunft ohne Nierenschädigung oder Lebererkrankung verglichen, die 1997 von einem der PROCAM-Studienzentren rekrutiert wurden (24).

### Laborverfahren:

Serum und EDTA-Plasma wurden nach 12 Stunden Fasten über Nacht genommen. Nach Zentrifugieren bei geringer Geschwindigkeit wurden die Proben eingefroren und vor der Analyse bei -80°C aufbewahrt (25). Je nach dem Serumkreatininwert wurden nach der Infusion von Iohexol während des selben Besuchs der Ambulanz zwei bis drei Blutproben zur Bestimmung der GFR mit Hilfe des Iohexolverfahrens (26) erhalten. Bei 18 Patienten mit meist fortgeschrittener Schädigung der Nierenfunktion wurde die GFR unter Verwendung der Formel von Cockcroft und Gault (27) errechnet. Die Patienten wurden sorgfältig über die Sammlung einer 24 Stunden Harnprobe zur Bestimmung der Proteinurie unterrichtet.

Die Messung von ApoA-IV, Lp(a), Serumalbumin, GFR, C-reaktivem Protein (CRP) und Apo(a)-Phänotypisierung erfolgten jeweils zentral bzw. in einem einzigen Labor, um Messunterschiede zwischen den Labors zu vermeiden. Zu dieser Zeit hatte das an dieser Studie mitarbeitende Laborpersonal keinen Einblick in die Nierenfunktion und Krankengeschichte der Patienten oder in die Einteilung der gemessenen Proben in Patient oder Kontrolle.

Die ApoA-IV-Konzentrationen im Plasma wurden mit Hilfe eines Festphasen-Enzymimmunoassays bestimmt, der affinitätsgereinigtes Hasen-anti-human-ApoA-IV-polyklonales Antiserum als Fangantikörper und den gleichen Antikörper, an Meerrettich-Peroxidase gekoppelt, als Nachweisantikörper verwendet (25, 28). Plasma mit einem bekannten Gehalt an ApoA-IV (standardisiert mit gereinigtem ApoA-IV nach der Phenylalanin-Quantifizierung mittels Hochdruckflüssigkeitschromatographie) diente als Kalibrierungsstandard. Die Intra-Assay- und Inter-Assay-Variationskoeffizienten dieses Assays betragen 4,5% bzw. 6,6% (25). Proben von Patienten und Kontrollen wurden doppelt innerhalb einer Serie als Bildprobe und nach einer gleich langen Lagerdauer der Proben bei -70°C analysiert. Die Lp(a)-Quantifizierung und die Apo(a)-Phänotypisierung erfolgten wie jüngst im Detail beschrieben (23). Das Serumalbumin (Bromkresolgrün-Verfahren) wurde mit Hilfe eines Kits von Roche (Basel, Schweiz) gemessen. Die Messungen erfolgten auf Mikrotiterplatten, wie früher beschrieben (25). CRP wurde auf einem Behring BNA-Nephelometer unter Verwendung der Reagenzien von Behring Diagnostics (N Latex CRP Mono; Behring Diagnostics, Marburg, Deutschland) gemessen. Die untere Nachweisgrenze dieses Tests betrug 0,02 mg/dl.

### Statistische Verfahren:

Die statistische Analyse wurde mit dem Statistical Package for the Social Sciences (SPSS) für Windows 10,0 durchgeführt. Univariahte Vergleiche kontinuierlicher Variablen zwischen den Kontrollen und den Nierenpatienten erfolgten durch den ungepaarten T-Test [unpaired t test] oder den nicht-parametrischen Rangsummentest nach Wilcoxon [non-parametric Wilcoxon rank sum test] im Falle von nicht normal verteilten Variablen. Dichotomisierte Variable wurden unter Verwendung von Pearson's c²-Test verglichen. ANOVA wurde verwendet, um kontinuierliche Variable zwischen Kontrollen und Nierenpatienten zu vergleichen, die an Hand der drei Tertilen der GFR in Untergruppen eingeteilt worden waren. Nicht normal verteilte Variable wurden logarithmisch transformiert, bevor sie in die Analyse einbezogen wurden. Der Korrelationstest nach Spearman wurde verwendet, um ApoA-IV mit anderen kontinuierlichen Variablen zu korrelieren. Die Anpassung der ApoA-IV-Serumkonzentrationen nach Alter und Proteinurie bei Patienten erfolgte mittels linearer Regressionsanalyse. Die multiple Regressionsanalyse wurde verwendet, um den Zusammenhang verschiedener Variablen mit den ApoA-IV-Serumkonzentrationen zu untersuchen. Die logistische Regressionsanalyse wurde durchgeführt, um Voranzeiger für prävalente atherosklerotische Ereignisse zu identifizieren, die in der Vergangenheit aufgetreten waren.

### Ergebnisse:

### Einfluss der Nierenfunktion auf ApoA-IV-Konzentrationen:

Nierenpatienten hatten signifikant erhöhte Gesamtcholesterin-, Triglycerid- und Lp(a)-Konzentrationen, jedoch unveränderte HDL- und LDL-Cholesterinwerte im Vergleich zu den Kontrollen (Tabelle 2). Die Konzentrationen von ApoA-I und ApoB waren bei den Patienten signifikant niedriger. Eine der deutlichsten Veränderungen gab es bei den ApoA-IV-Konzentrationen, die bei Patienten um fast 70% höher waren als bei den Kontrollen (24,6 ± 8,6 vs. 14,6 ± 4,2 mg/dl, P < 0,001).

**Tabelle 2:**

| **Serumkonzentrationen von Lipiden, Lipoproteinen und Apolipoproteinen bei Kontrollen und Patienten mit Nierenerkrankung ^{a}** | | |
|---|---|---|
| | **Kontrollen (n = 227)** | **Nierenpatienten (n = 227)** |
| Gesamtcholesterin, mg/dl | 206 ± 41 | 215 ± 45 ^{b} |
| HDL-Cholesterin, mg/dl | 43,6 ± 11,5 | 43,7 ± 14,2 |
| LDL-Cholesterin, mg/dl | 134 ± 37 | 136 ± 40 |
| Triglyceride, mg/dl; mittl. SD | 141 ± 96 | 173 ± 101 ^{c} |
| [25. Percentil, Mittel, 75. Percentil] | [84, 114, 170] | [101, 144, 223] |
| Apolipoprotein A-I, mg/dl | 157 ± 22 | 120 ± 21 ^{c} |
| Apolipoprotein A-IV, mg/dl | 14,6 ± 4,2 | 24,6 ± 8,6 ^{c} |
| Apolipoprotein B, mg/dl | 123 ± 30 | 107 ± 27 ^{c} |
| Lipoprotein(a), mg/dl; mittl. ± SD | 20,7 ± 32,8 | 29,5 ± 32,0 ^{c} |
| [25. Percentil, Mittel, 75. Percentil] | [2,2, 6,9, 19,4] | [4,9, 17,9, 42,5] |

| | | |
|---|---|---|
| ^{a} Daten sind Mittel ± SD und, wo zutreffend, [25. Percentil, Mittel, 75. Percentil]. ^{b} P < 0,05 für den Vergleich mit den Testpersonen der Kontrolle. ^{c} P < 0,001 für den Vergleich mit den Testpersonen der Kontrolle. | | |

Die Berechnung der Korrelationskoeffizienten zwischen ApoA-IV und mehreren Variablen bei Patienten ergab die höchsten Korrelationen mit Parametern der Nierenfunktion, wobei sich höhere Konzentrationen von ApoA-IV mit sinkender Nierenfunktion ergaben (Serumkreatinin r = 0,73, Serumharnstoff r = 0,66 und GFR r = -0,62, tägliche Proteinurie r = 0,37) (Tabelle 3 und Fig. 1). Wesentlich geringere Korrelationen wurden mit dem Alter (r = 0,20), dem Gesamtcholesterin (r = 0,13), ApoA-I (r = 0,14) und Lp(a) (r = 0,14) beobachtet. Als die Konzentrationen von ApoA-IV nach Alter und Proteinurie angepasst wurden, wurden noch immer starke Korrelationen mit der Nierenfunktion, jedoch keine Korrelationen mit Variablen vom Lipoproteinmetabolismus beobachtet.

**Tabelle 3:**

| **Korrelationen der Serumkonzentrationen von Apolipoprotein A-IV mit ausgewählten variablen.** | | |
|---|---|---|
| **Variable** | **ApoA-IV Rohwerte** | **ApoA-IV kontrolliert für Alter und Proteinurie** |
| Alter | 0,20^{b} | -- |
| Körpermasseindex | 0,00 | -0,06 |
| Kreatinin | 0,73^{c} | 0,56^{c} |
| Harnstoff | 0,66^{c} | 0,56^{c} |
| GFR | -0,62^{c} | -0,54^{c} |
| Proteinurie | 0,37^{c} | -- |
| Serumalbumin | -0,09 | 0,05 |
| CRP | -0,05 | -0,16^{a} |
| Gesamtcholesterin | 0,13^{a} | 0,10 |
| HDL-Cholesterin | 0,03 | 0,02 |
| LDL-Cholesterin | 0,13 | 0,11 |
| Triglyceride | 0,05 | -0,02 |
| Apolipoprotein A-I | 0,14^{a} | 0,12 |
| Apolipoprotein B | 0,04 | 0,01 |
| Lipoprotein(a) | 0,14^{a} | 0,07 |

| | | |
|---|---|---|
| ^{a} P < 0,05; ^{b} P < 0,01; ^{c} p < 0,001 | | |

Es wurden die ApoA-IV-Konzentrationen in 3 Bereichen der Nierenfunktion errechnet, um zu untersuchen, in welcher Phase der Nierenschädigung der Anstieg von ApoA-IV beginnt (Tabelle 4). Dafür wurden die Nierenpatienten nach den Tertilen der GFR gruppiert, d.h. > 90 ml/min/1,73 m², 45 - 90 ml/min/1,73 m² und < 45 ml/min/ 1,73 m². Die Konzentrationen von ApoA-IV stiegen mit der sinkenden Nierenfunktion signifikant an (P < 0,001 mit ANOVA), und ApoA-IV war in der Gruppe von Patienten mit primärer Nierenerkrankung bereits erhöht, die jedoch im Vergleich zu den Kontrollen noch fast normale GFR-Werte (> 90 ml/min/1,73m²) hatten (17,7 ± 6,2 vs. 14,6 ± 4,2 mg/dl, P < 0,001). Diese Befunde änderten sich nicht, wenn die ApoA-IV-Konzentrationen der Patienten nach Alter und Proteinurie angepasst wurden. Es wurde auch bestimmt, ob die primäre Ursache der Nierenerkrankung die Konzentrationen von ApoA-IV beeinflusst. Patienten mit polyzystischer Nierenerkrankung zeigten eine Tendenz zu leicht höheren ApoA-IV-Konzentrationen im Vergleich zu jenen mit Glomerulonephritis (25,6 ± 8,8 vs. 23,0 ± 8,1 mg/dl, P = 0,11). Als die ApoA-IV-Konzentrationen jedoch auf die Unterschiede bei GFR und Proteinurie angepasst wurden, zeigten diese beiden Patientengruppen keine Unterschiede in den ApoA-IV-Werten mehr (24,5 ± 6,7 vs. 23,5 ± 7,1 mg/dl, P = 0,48).

**Tabelle 4:**

| **Mittel ± SD ApoA-IV-Serumkonzentrationen bei Kontrollen und Patienten mit Nierenerkrankung** | | |
|---|---|---|
| | **ApoA-IV, mg/dl Rohwerte** | **ApoA-IV, mg/dl angepasst nach Alter und Proteinurie** |
| Kontrollen (n = 227) | 14,6 ± 4,2 | - |
| GFR > 90 ml/min/1,73 m² (n = 72) | 17,7 ± 6,2 | 18,4 ± 6,1 |
| GFR 45-90 ml/min/1,73m² (n = 76) | 25,1 ± 7,9 | 24,7 ± 7,3 |
| GFR < 45 ml/min/1,73 m² (n = 79) | 30,5 ± 6,2 | 30,1 ± 6,2 |
| P-Wert von ANOVA | < 0,001 ^{a} | < 0,001 ^{a} |

| | | |
|---|---|---|
| ^{a}Post-hoc-Vergleiche zwischen allen möglichen Gruppenpaaren zeigten P-Werte von < 0,001 (nach Korrektur gemäß dem Verfahren von Bonferroni). | | |

In einem nächsten Schritt wurde mittels multipler Regressionsanalyse analysiert, welche Variablen bei Nierenpatienten in signifikantem Zusammenhang mit den ApoA-IV-Konzentrationen stehen. Da GFR, Serumkreatinin und Serumharnstoff stark korrelierten, wurden 3 verschiedene Modelle berechnet, die sehr ähnliche Ergebnisse erbrachten. ApoA-IV zeigte den stärksten Zusammenhang mit den Parametern der Nierenfunktion (GFR oder Serumkreatinin oder Serumharnstoff), was etwa 35% der ApoA-IV-Konzentrationen erklärte. Weitere, jedoch wesentlich geringere Beiträge zu den ApoA-IV-Konzentrationen kamen von der Proteinurie und ApoA-I (Tabelle 5).

**Tabelle 5:**

| **Der Zusammenhang von Variablen mit Serumkonzentrationen von Apolipoprotein A-IV bei Patienten mit geringem oder mäßigem Nierenversagen, bestimmt durch multiple Regressionsanalyse.** | | | | |
|---|---|---|---|---|
| **Variable** | **Koeffizient** | **SE** | **P** | **Veränderung in R²** |
| Modell 1 (GFR) | | | | |
| GFR . | -0,114 | 0,011 | < 0,001 | 0,354 |
| ln Proteinurie | 1,265 | 0,334 | < 0,001 | 0,044 |
| Apolipoprotein A-I | 0,050 | 0,020 | 0,012 | 0,017 |
| Modell 2 (Kreatinin) | | | | |
| Kreatinin | 4,167 | 0,391 | < 0,001 | 0,350 |
| ln Proteinurie | 1,207 | 0,333 | < 0,001 | 0,043 |
| Apolipoprotein A-I | 0,063 | 0,020 | 0,002 | 0,027 |
| Modell 3 (Harnstoff) | | | | |
| Harnstoff | 0,143 | 0,013 | < 0,001 | 0,362 |
| ln Proteinurie | 1,151 | 0,334 | 0,001 | 0,038 |
| Apolipoprotein A-I | 0,058 | 0,020 | 0,003 | 0,023 |

Variable und Interaktionsbedingungen, die nicht signifikant zum multiplen Regressionsmodell beitrugen: Gesamtcholesterin, HDL-und LDL-Cholesterin, Triglyceride, Apolipoprotein B, Lipoprotein (a), Serumalbumin, Rauchen, Körpermasseindex, Alter, Geschlecht und die Interaktionsbedingungen GFR*Proteinurie, Kreatinin*Proteinurie und Harnstoff*Proteinurie. "ln Proteinurie" bedeutet, dass die Variable logarithmisch transformiert ist.

Koeffizienten und SE für GFR, Kreatinin und Harnstoff sind zur besseren Interpretation in natürlichem Maßstab angegeben. Die Verwendung logarithmisch transformierter Werte ergab ähnliche Beiträge dieser Variablen zu den Modellen.

### ApoA-IV und atherosklerotische Komplikationen:

Schließlich wurde ein Zusammenhang zwischen den ApoA-IV-Konzentrationen und vorhandenen atherosklerotischen Komplikationen analysiert. 26 Patienten hatten bereits 36 atherosklerotische Ereignisse erlitten, darunter 17 koronare Ereignisse (hauptsächlich Myokardinfarkte und aortokoronäre Bypasse), 9 Schlaganfälle und 10 Ereignisse, die das periphere Arteriensystem betrafen. Patienten mit atherosklerotischen Komplikationen zeigten zumindest eine Tendenz zu niedrigeren ApoA-IV-Konzentrationen in allen drei Bereichen der GFR im Vergleich zu den Patienten ohne Komplikationen (Tabelle 6). Höchstwahrscheinlich auf Grund der kleinen Probengröße in jedem einzelnen Bereich war der Unterschied nicht in jeder Gruppe signifikant. In der Analyse wurde auch eine logistische Regressionsanalyse für die gesamte Patientengruppe einschließlich GFR durchgeführt. Das sparsamste Modell identifizierte drei Variable, die mit atherosklerotischen Komplikationen in Zusammenhang stehen: Alter, ApoA-IV und Geschlecht (Tabelle 7). Jede 1 mg/dl Steigerung von ApoA-IV senkte die Wahrscheinlichkeit für eine atherosklerotische Komplikation um 8% (P = 0,011). Der LMW Apo(a) Phänotyp und GFR zeigten einen Grenzzusammenhang mit atherosklerotischen Komplikationen (Tabelle 7).

**Tabelle 6:**

| **Mittlere (± SD) Serumkonzentrationen von ApoA-IV bei Patienten mit und ohne atherosklerotische Ereignisse für die drei Bereiche der glomerulären Filtrationsrate (GFR)** | | | |
|---|---|---|---|
| | **ohne Ereignisse (n = 201)** | **mit Ereignissen (n = 26)** | **P** |
| GFR > 90 ml/min/1,73 m² | 18,0 ± 6,3 | 14,0 ± 4,0 | 0,17 |
| (n = 72) | (n = 67) | (n = 5) | |
| GFR 45-90 ml/min/1,73 m² | 25,6 ± 8,1 | 21,4 ± 5,5 | 0,12 |
| (n = 76) | (n = 66) | (n = 10) | |
| GFR < 45 ml/min/1,73 m² | 31,1 ± 5,8 | 27,0 ± 7,5 | 0,04 |
| (n = 79) | (n = 68) | (n = 11) | |
| Alle Patienten | 24,9 ± 8,7 | 22,3 ± 7,7 | 0,15 |
| | (n = 201) | (n = 26) | |

**Tabelle 7:**

| **Logistische Regressionsanalyse zur Untersuchung der Vorhersagewerte von ApoA-IV und anderen Variablen für atherosklerotische Ereignisse.** | | | | | | |
|---|---|---|---|---|---|---|
| **Variable (Zunahme)** | **Koeffizient** | **SEM** | **c²** | **OR** | **(95% CI)** | **P** |
| Alter | 0,080 | 0,027 | 9,1 | 1,08 | (1,03-1,14) | 0,003 |
| ApoA-IV | -0,085 | 0,033 | 6,5 | 0,92 | (0,86-0,98) | 0,011 |
| Geschlecht | 1,434 | 0,655 | 4,8 | 4,20 | (1,16-15,15) | 0,028 |

Variable nicht im Modell: LMW Apo(a) Phänotyp (P = 0,11) und lntransformierte GFR (P = 0,16).

### Diskussion:

### Einfluss der Nierenfunktion auf die ApoA-IV-Konzentrationen:

Die vorliegende Studie zeigt einen starken Einfluss der Nierenfunktion auf die ApoA-IV-Serumkonzentrationen. Frühere Studien zeigten, dass Patienten mit einer Nierenerkrankung im Endstadium einen deutlichen Anstieg der ApoA-IV-Konzentrationen aufweisen (18-20, 22). Es war jedoch völlig unbekannt, dass bereits eine geringfügige Schädigung durch eine Nierenerkrankung mit fast normaler GFR von einem signifikanten Anstieg der ApoA-IV-Konzentrationen begleitet ist. Es scheint daher, dass ApoA-IV ein früher Marker für eine Nierenschädigung ist. Dies könnte auch der Grund dafür sein, dass eine jüngste Studie von Sun et al. bei Männern und Frauen mittleren und höheren Alters Diabetes mellitus als eine Hauptdeterminante der ApoA-IV-Konzentrationen identifizierte (29). Da viele Patienten mit Diabetes mellitus eine Schädigung der Nierenfunktion haben, und da bereits eine sehr geringfügige Schädigung der Nierenfunktion mit einem Anstieg von ApoA-IV assoziiert ist, wird angenommen, dass eher die Nierenfunktion als Diabetes mellitus an sich für den Anstieg von ApoA-IV verantwortlich ist. Dies wird weiters von den vorliegenden Daten gestützt, da Diabetes mellitus bei der vorliegenden Studie ein Ausschlusskriterium war, und es dennoch zu einem Anstieg von ApoA-IV kam. Darüber hinaus sind in einer früheren Studie bei Patienten mit Nierenerkrankungen im Endstadium keine Unterschiede in den ApoA-IV-Werten beobachtet worden, als 189 Patienten mit Diabetes mellitus mit 513 nicht diabetischen Patienten verglichen wurden (22). Andere Studien an Patienten mit Diabetes mellitus berichteten jedoch von einem Anstieg von ApoA-IV bei diesen Patienten ohne sorgfältige Inbetrachtnahme der Nierenfunktion (30, 31). Vergès et al. schlossen nur Patienten mit Nierenversagen aus, ohne genaue Informationen über die Ausschlusskriterien zu geben (30, 31). Etwa 40% der Patienten hatten jedoch Mikroalbuminurie (31), was zumindest auf eine Beteiligung der Niere hinweist, von der zu erwarten ist, dass sie die ApoA-IV-Konzentrationen signifikant beeinflusst.

Neben Diabetes mellitus berichtete die Studie von Sun und Kollegen auch von einer signifikanten Korrelation zwischen Alter und ApoA-IV-Konzentrationen (29), die hauptsächlich durch die Nierenfunktion erklärt werden kann. Es ist wohl bekannt, dass das Altern mit einem enormen Verlust an Nephronen und der glomerulären Filtrationsrate einhergeht. Eine 70 Jahre alte Person hat eine GFR von nur etwa 60-70% eines jungen Erwachsenen. Daher ist die GFR bereits in vielen Fällen in einem Bereich wie im mittleren GFR-Tertil unserer Patienten, die bereits einen deutlichen Anstieg der ApoA-IV-Werte aufwiesen. Als ApoA-IV in der von der PROCAM-Studie rekrutierten Kontrollgruppe mit dem Alter korreliert wurde, wurde keine signifikante Korrelation beobachtet (r = 0,057, P = 0,39). Dies kann einfach durch die Tatsache erklärt werden, dass eine große Anzahl von Kontrollen mit Nierenschäden von der Analyse ausgeschlossen wurde, indem ein Serumkreatinin von > 1,5 mg/dl und/oder Makroalbuminurie als Ausschlusskriterien genommen wurden. Dies weist auf die Notwendigkeit einer sorgfältigen Kontrolle der ApoA-IV-Konzentrationen für die Nierenfunktion in Fall-Kontroll-Studien hin. Wenn die Nierenfunktion nicht in Betracht gezogen wird, kann die Interpretation von Ergebnissen irreführend sein, wo es Unterschiede in der Nierenfunktion zwischen den Testpersonen und den Kontrollen gibt. Die Folge können entweder falsche positive oder falsche negative Assoziationen sein, je nach der Verteilung der Nierenfunktion bei den Testpersonen und den Kontrollen. Es ist eine offene Frage, ob ApoA-IV bei Nierenproblemen voll funktionsfähig ist. Es könnte angenommen werden, dass Urämie einen Einfluss z.B. auf die enzymaktivierenden und antioxidativen Eigenschaften von ApoA-IV hat. Eine Erhöhung von ApoA-IV, um die Funktionsstörung zu kompensieren, ist jedoch nicht wahrscheinlich. Es scheint wohl sehr viel wahrscheinlicher, dass die Erhöhung von ApoA-IV sekundär ist, bedingt durch eine katabolische Blockade in der Niere, was von Beobachtungen bei Ratten gestützt wird, dass ApoA-IV von Niere und Leber katabolisiert wird. Die histologische Analyse zeigte, dass ApoA-IV innerhalb proximaler Tubularzellen und im tubulären Lumen lokalisiert ist (32). Wegen seines Molekulargewichts von etwa 46 kDa kann ApoA-IV von Glomeruli zumindest in seiner Lipoproteinungebundenen (freien) Form filtriert werden. Auf die Aufnahme durch proximale Tubularzellen könnte dann der Abbau folgen. Es muss jedoch noch festgestellt werden, ob der katabolische Weg bei Menschen der gleiche ist wie bei Ratten.

### ApoA-IV und atherosklerotische Komplikationen:

In jüngster Zeit sind zum ersten Mal signifikant niedrigere ApoA-IV-Werte bei 114 männlichen, weißhäutigen Testpersonen mit angiographisch definiertem CAD im Vergleich zu 114 in ihrem Alter angepassten männlichen Kontrollen berichtet worden (10,2 ± 3,8 mg/dl vs. 15,1 ± 4,0 mg/dl, p < 0,001). Diese inverse Beziehung zwischen den ApoA-IV-Werten und dem Vorhandensein eines CAD wurde in einer unabhängigen Probe von 68 männlichen Indern mit angiographisch dokumentiertem CAD und 68 im Alter angepassten Kontrollen bestätigt. Entsprechend diesem Befund wurden bei Patienten, die bereits ein atherosklerotisches Ereignis hinter sich hatten, im Vergleich zu den Kantrollen, bei denen dies nicht der Fall war, niedrigere ApoA-IV-Konzentrationen beobachtet. Dies wurde in allen drei Bereichen der GFR beobachtet, es wurde jedoch nur im Bereich mit der schlechtesten GFR das herkömmliche Signifikanzniveau erreicht, was durch die geringe Anzahl von Patienten mit Ereignissen in einem einzelnen Bereich erklärt werden könnte (Tabelle 6). Als die ganze Gruppe in der logistischen Regressionsanalyse in Betracht gezogen wurde, wobei GFR dem statistischen Modell geboten wurde, erwies sich ApoA-IV unabhängig von der GFR als ein verlässliches Vorzeichen für Ereignisse (Tabelle 7). Die Beweise, die diesen Zusammenhang sehr wahrscheinlich machen, sind stark: Zellkulturstudien, die eine Beteiligung von ApoA-IV bei mehreren Schritten des reversen Cholesterintransportes (9-14) sowie antioxidative Eigenschaften zeigen (33); Überexpression von ApoA-IV von Menschen und Mäusen in Mäusen führte zu einem Sinken der Atherosklerose (16, 17); und schließlich erste Ergebnisse von niedrigeren ApoA-IV-Konzentrationen bei Männern mit angiographisch nachgewiesenem CAD (34).

Die Ergebnisse dieser und einer anderen Studie (34) stehen im Gegensatz zu einer Untersuchung an Patienten mit nicht-insulinabhängigem Diabetes mellitus, worin signifikant höhere ApoA-IV-Konzentrationen bei Patienten im Vergleich zu jenen ohne makrovaskuläre Komplikationen beschrieben sind (31). Die Erklärung für diese Diskrepanz könnte das erhöhte Vorkommen von Mikroalbuminurie und die damit einhergehende Nierenschädigung bei diabetischen Patienten mit makrovaskulären Komplikationen sein. Daher können höhere Werte von ApoA-IV bei diabetischen Patienten mit makrovaskulären Komplikationen einfach ihre geschädigte Nierenfunktion widerspiegeln.

Im Moment ist zu wenig über die Regulierung von ApoA-IV-Serumkonzentrationen bekannt, was für die Entwicklung einer therapeutischen Intervention verwendet werden könnte. Sobald therapeutische Mittel entwickelt worden sind, die die ApoA-IV-Konzentrationen erhöhen, werden sie als eines der überzeugendsten Experimente für die Untersuchung der Pathogenität von ApoA-IV verwendet werden.

Man könnte sich fragen, warum Patienten mit Nierenerkrankungen ein solch hohes Risiko für atherosklerotische Komplikationen haben (35), wenn sie doch so hohe ApoA-IV-Konzentrationen haben. Wenn ApoA-IV tatsächlich als Antiatherogen wirkt, sollten diese Patienten besser geschützt sein als andere. Dieses Argument ist von einem univarianten Standpunkt aus gesehen recht verführerisch. Man sollte dabei jedoch nicht vergessen, dass bei diesen Patienten mehrere Risikofaktoren für Atherosklerose erheblich in Richtung eines ungünstigen Profils verschoben sind (35). Neben den Veränderungen bei den traditionellen Risikofaktoren für Atherosklerose sind jüngst selbst in Stadien, wo die Nierenerkrankung nur mit geringen Veränderungen der GFR einhergeht (23, 38, 39), deutliche Veränderungen für Lp(a) und Homocystein (für einen Überblick siehe (36, 37)) gezeigt worden. Mehrere Studien zeigten, dass sowohl Homocystein als auch Lp(a) und insbesondere Lp(a) von LMW Apo(a) Phänotypen bei diesen Patienten wichtige Risikofaktoren für Atherosklerose sind (40-48). Es ist noch nicht erwiesen, ob ApoA-IV gegen diese Belastung wirkt, dies wird jedoch von den niedrigeren ApoA-IV-Werten bei jenen mit atherosklerotischen Problemen gestützt.

Es ist eine interessante Beobachtung, dass die gegenwärtigen Patienten normale HDL-Cholesterinwerte, jedoch deutlich verminderte ApoA-I-Werte hatten. Andere Studien untersuchten üblicher Weise Patienten mit Nierenschäden in einem fortgeschritteneren Stadium mit niedrigeren GFR-Konzentrationen und beschrieben verminderte HDL-Cholesterinkonzentrationen (49-51). Im Gegensatz zu diesen Untersuchungen zeigten die gegenwärtigen Patienten eine höhere durchschnittliche GFR von etwa 70 ml/min/1,73 m², was darauf hindeutet, dass dem Sinken des HDL-Cholesterins eine ApoA-I-Erschöpfung oder eine Akkumulation von Lipidpartikeln von HDL-Cholesterin vorausgeht, was ein Anzeichen für einen gestörten reversen Cholesterintransportweg sein könnte. Da ApoA-I und ApoA-IV eine wesentliche funktionelle Überlappung (z.B. LCAT-Aktivierung) aufweisen, muss noch nachgewiesen werden, ob eine Erhöhung von ApoA-IV ein Versuch ist, das Absinken von ApoA-I zu kompensieren.

Insgesamt zeigen die vorliegenden Daten deutlich, dass ApoA-IV während der frühesten Phasen einer Niereninsuffizienz zu steigen beginnt, was ApoA-IV zu einem frühen Marker für eine Nierenschädigung macht. Weiters scheint ApoA-IV bei Patienten mit geringfügigem bis mäßigem Nierenversagen mit atherosklerotischen Komplikationen in Zusammenhang zu stehen.

### Literatur:

1. Utermann G, Beisiegel U: Apolipoprotein A-IV: a protein occurring in human mesenteric lymph chylomicrons and free in plasma. Isolation and quantification. Eur J Biochem 99:333-343, 1979.
2. Green PHR, Glickman RM, Riley JW, Qinet E: Human apolipoprotein A-IV. Intestinal origin and distribution in plasma. J Clin Invest 65:911-919, 1980.
3. Duverger N, Ghalim N, Ailhaud G, Steinmetz A, Fruchart J-C, Castro G: Characterisation of apoA-IV-containing lipoprotein particles isolated from human plasma and interstitial fluid. Arterioscler Thromb 13:126-132, 1993.
4. Von Eckardstein A, Huang Y, Wu S, Sarmadi AS, Schwarz S, Steinmetz A, Assmann G: Lipoproteins containing apolipoprotein A-IV but not apolipoprotein A-I take up and esterify cell-derived cholesterol in plasma. Arterioscler Thromb Vasc Biol 15:1755-1763, 1995.
5. Apfelbaum TF, Davidson NO, Glickman RM: Apolipoprotein A-IV synthesis in rat intestine: regulation by dietary triglyceride. Am J Physiol 252:G662-G6661987.
6. Fujimoto K, Cardelli JA, Tso P: Increased apolipoprotein A-IV in rat mesenteric lymph after lipid meal acts as a physiological signal for satiation. Am J Physiol 262:G1002-G10061992.
7. Aalto-Setälä K, Bisgaier CL, Ho A, Kieft KA, Traber MG, Kayden HJ, Ramakrishnan R, Walsh A, Essenburg AD, Breslow JL: Intestinal expression of human apolipoprotein A-IV in transgenic mice fails to influence dietary lipid absorption or feeding behavior. J Clin Invest 93:1776-1786, 1994.
8. Weinstock PH, Bisgaier CL, Hayek T, Aalto-Setala K, Sehayek E, Wu L, Sheiffele P, Merkel M, Essenburg AD, Breslow JL: Decreased HDL cholesterol levels but normal lipid absorption, growth, and feeding behavior in apolipoprotein A-IV knockout mice. J Lipid Res 38:1782-1794, 1997.
9. Stein o, Stein Y, Lefevre M, Roheim PS: The role of apolipoprotein A-IV in reverse cholesterol transport studied with cultured cells and liposomes derived from another analog of phosphatidylcholine. Biochim Biophys Acta 878:7-13, 1986.
10. Dvorin E, Gorder NL, Benson DM, Gotto Jr. AM: Apolipoprotein A-IV. A determinant for binding and uptake of high density lipoproteins by rat hepatocytes. J Biol Chem 261:15714-15718, 1986.
11. Steinmetz A, Barbaras R, Ghalim N, Clavey V, Fruchart J-C, Ailhaud G: Human apolipoprotein A-IV binds to apolipoprotein A-I/A-II receptor sites and promotes cholesterol efflux from adipose cells. J Biol Chem 265:7859-7863, 1990.
12. Steinmetz A, Utermann G: Activation of lecithin:cholesterol acyltransferase by human apolipoprotein A-IV. J Biol Chem 260:2258-2264, 1985.
13. Chen CH, Albers JJ: Activation of lecithin:cholesterol acyltransferase by apolipoproteins E-2, E-3 and A-IV isolated from human plasma. Biochim Biophys Acta 836:279-285, 1985.
14. Goldberg IJ, Scheraldi CA, Yacoub LK, Saxena U, Bisgaier CL: Lipoprotein ApoC-II activation of lipoprotein lipase. Modulation by apolipoprotein A-IV. J Biol Chem 265:4266-4272, 1990.
15. Guyard-Dangremont V, Lagrost L, Gambert P: Comparative effects of purified apolipoproteins A-I, A-II, and A-IV on cholesteryl ester transfer protein activity. J Lipid Res 35:982-992, 1994.
16. Duverger N, Tremp G, Caillaud JM, Emmanuel F, Castro G, Fruchart JC, Steinmetz A, Denèfle P: Protection against atherogenesis in mice mediated by human apolipoprotein A-IV. Science 273:966-968, 1996.
17. Cohen RD, Castellani LW, Qiao JH, Van Lenten BJ, Lusis AJ, Reue K: Reduced aortic lesions and elevated high density lipoprotein levels in transgenic mice overexpressing mouse apolipoprotein A-IV. J Clin Invest 99:1906-1916, 1997.
18. Nestel PJ, Fide NH, Tan MH: Increased lipoprotein-remnant formation in chronic renal failure. N Engl J Med 307:329-333, 1982.
19. Seishima M, Muto Y: An increased apo A-IV serum concentration of patients with chronic renal failure on hemodialysis. Clin Chim Acta 167:303-311, 1987.
20. Dieplinger H, Lobentanz E-M, König P, Graf H, Sandholzer C, Matthys E, Rosseneu M, Utermann G: Plasma apolipoprotein A-IV metabolism in patients with chronic renal disease. Eur J Clin Invest 22:166-174, 1992.
21. Dieplinger H, Schoenfeld PY, Fielding J: Plasma cholesterol metabolism in end-stage renal disease: difference between treatment by hemodialysis or peritoneal dialysis. J Clin Invest 77:1071-1083, 1986.
22. Kronenberg F, König P, Neyer U, Auinger M, Pribasnig A, Lang U, Reitinger J, Pinter G, Utermann G, Dieplinger H: Multicentre study of lipoprotein(a) and apolipoprotein(a) phenotypes in patients with end-stage renal disease treated by hemodialysis or continuous ambulatory peritoneal dialysis. J Am Soc Nephrol 6:110-120, 1995.
23. Kronenberg F, Kuen E, Ritz E, Junker R, König P, Kraatz G, Lhotta K, Mann JFE, Müller GA, Neyer U, Riegel W, Riegler P, Schwenger V, Von Eckardstein A: Lipoprotein(a) serum concentrations and apolipoprotein (a) phenotypes in mild and moderate renal failure. J Am Soc Nephrol 11:105-115, 2000.
24. Assmann G, Schulte H, Von Eckardstein A: Hypertriglyceridemia and elevated lipoprotein(a) are risk factors for major coronary events in middle-aged men. Am J Cardiol 77:1179-1184, 1996.
25. Kronenberg F, Lobentanz E-M, König P, Utermann G, Dieplinger H: Effect of sample storage on the measurement of lipoprotein(a), apolipoproteins B and A-IV, total and high-density lipoprotein cholesterol and triglycerides. J Lipid Res 35:1318-1328, 1994.
26. Gaspari F, Perico N, Matalone M, Signorini O, Azzollini N, Mister M, Remuzzi G: Precision of plasma clearance of iohexol for estimation of GFR in patients with renal disease. J Am Soc Nephrol 9:310-313, 1998.
27. Cockcroft DW, Gault MH: Prediction of creatinine clearance from serum creatinine. Nephron 16:31-41, 1976.
28. Rosseneu M, Michiels G, Dekeersgieter W, Bury JB, De Slypere JP, Dieplinger H, Utermann G: Human apolipoprotein A-IV quantitation by sandwich enzyme linked immunosorbent assay. Clin Chem 34:739-743, 1988.
29. Sun Z, Larson IA, Ordovas JM, Barnard JR, Schaefer EJ: Effects of age, gender, and lifestyle factors on plasma apolipoprotein A-IV concentrations. Atherosclerosis 151:381-388, 2000.
30. Vergès BL, Vaillant G, Goux A, Lagrost L, Brun JM, Gambert P: Apolipoprotein A-IV levels and phenotype distribution in NIDDM. Diabetes Care 17:810-817, 1994.
31. Vergès BL, Lagrost L, Vaillant G, Petit JM, Cohen M, Gambert P, Brun JM: Macrovascular disease is associated with increased plasma apolipoprotein A-IV levels in NIDDM. Diabetes 46:125-132, 1997.
32. Dallinga-Thie GM, Van't Hooft FM, van Tol A: Tissue sites of degradation of high density lipoprotein apolipoprotein A-IV in rats. Arteriosclerosis 6:277-284, 1986.
33. Qin XF, Swertfeger DK, Zheng SQ, Hui DY, Tso P: Apolipoprotein AIV: A potent endogenous inhibitor of lipid oxidation. Am J Physiol 274:H1836-H18401998.
34. Kronenberg F, Stühlinger M, Trenkwalder E, Geethanjali FS, Pachinger O, Von Eckardstein A, Dieplinger H: Low apolipoprotein A-IV plasma concentrations in men with coronary artery disease. J Am Coll Cardiol 36:751-757, 2000.
35. London GM, Drüeke TB: Atherosclerosis and arteriosclerosis in chronic renal failure. Kidney Int 51:1678-1695, 1997.
36. Bostom AG, Lathrop L: Hyperhomocysteinemia in end-stage renal disease: Prevalence, etiology, and potential relationship to arteriosclerotic outcomes. Kidney Int 52:10-20, 1997.
37. Kronenberg F: Homocysteine, lipoprotein(a) and fibrinogen: metabolic risk factors for cardiovascular complications of chronic renal disease. Curr Opin Nephrol Hypertens 7:271-278, 1998.
38. Bostom AG, Kronenberg F, Jacques PF, Kuen E, Ritz E, König P, Kraatz G, Lhotta K, Mann JFE, Müller GA, Neyer U, Riegel W, Schwenger V, Riegler P, Selhub J: Proteinuria and plasma total homocysteine levels in chronic renal disease patients with a normal range serum creatinine: critical impact of true glomerular filtration rate. Atherosclerosis (in press).
39. Bostom AG, Kronenberg F, Gohh RY, Schwenger V, Kuen E, König P, Kraatz G, Lhotta K, Mann JFE, Müller GA, Neyer U, Riegel W, Riegler P, Ritz E, Selhub J: Chronic renal transplantation: a model for the hyperhomocysteinemia of renal insufficiency. Atherosclerosis 156:227-230, 2001.
40. Jungers P, Massy ZA, Khoa TN, Fumeron C, Labrunie N, Lacour B, Descamps-Latscha B, Man NK: Incidence and risk factors of atherosclerotic cardiovascular accidents in predialysis chronic renal failure patients: a prospective study. Nephrol Dial Transplant 12:2597-2602, 1997.
41. Bachmann J, Tepel M, Raidt H, Riezler R, Graefe U, Langer K, Zidek W: Hyperhomocysteinemia and the risk for vascular disease in hemodialysis patients. J Am Soc Nephrol 6:121-125, 1995.
42. Robinson K, Gupta A, Dennis V, Arheart K, Chaudhary D, Green R, Vigo P, Mayer EL, Selhub J, Kutner M, Jacobsen DW: Hyperhomocysteinemia confers an independent increased risk of atherosclerosis in end-stage renal disease and is closely linked to plasma folate and pyridoxine concentrations. Circulation 94:2743-2748, 1996.
43. Bostom AG, Shemin D, Verhoef P, Nadeau MR, Jacques PF, Selhub J, Dworkin L, Rosenberg IH: Elevated fasting total plasma homocysteine levels and cardiovascular disease outcomes in maintenance dialysis patients. A prospective study. Arterioscler Thromb Vasc Biol 17:2554-2558, 1997.
44. Moustapha A, Naso A, Nahlawi M, Gupta A, Arheart KL, Jacobsen DW, Robinson K, Dennis VW: Prospective study of hyperhomocysteinemia as an adverse cardiovascular risk factor in end-stage renal disease. Circulation 97:138-141, 1998.
45. Cressman MD, Heyka RJ, Paganini EP, O'Neil J, Skibinski CI, Hoff HF: Lipoprotein(a) is an independent risk factor for cardiovascular disease in hemodialysis patients. Circulation 86:475-482, 1992.
46. Kronenberg F, Kathrein H, König P, Neyer U, Sturm W, Lhotta K, Gröchenig E, Utermann G, Dieplinger H: Apolipoprotein(a) phenotypes predict the risk for carotid atherosclerosis in patients with end-stage renal disease. Arterioscler Thromb 14:1405-1411, 1994.
47. Koch M, Kutkuhn B, Trenkwalder E, Bach D, Grabensee B, Dieplinger H, Kronenberg F: Apolipoprotein B, fibrinogen, HDL cholesterol and apolipoprotein(a) phenotypes predict coronary artery disease in hemodialysis patients. J Am Soc Nephrol 8:1889-1898, 1997.
48. Kronenberg F, Neyer U, Lhotta K, Trenkwalder E, Auinger M, Pribasnig A, Meisl T, König P, Dieplinger H: The low molecular weight apo(a) phenotype is an independent predictor for coronary artery disease in hemodialysis patients: a prospective follow-up. J Am Soc Nephrol 10:1027-1036, 1999.
49. Attman P-O, Alaupovic P: Lipid and apolipoprotein profiles of uremic dyslipoproteinemia. Relation to renal function and dialysis. Nephron 57:401-410, 1991.
50. Attman PO, Alaupovic P, Gustafson A: Serum apolipoprotein profile of patients with chronic renal failure. Kidney Int 32:368-375, 1987.
51. Attman PO, Alaupovic P, Tavella M, Knight-Gibson C: Abnormal lipid and apolipoprotein composition of major lipoprotein density classes in patients with chronic renal failure. Nephrol Dial Transplant 11:63-69, 1996.

## Patentansprüche

1. Verfahren zur Diagnose von Nierenschäden im Frühstadium bei einem Menschen, **gekennzeichnet durch** die folgenden Schritte:
- Messen der Menge von ApoA-IV in einer Körperflüssigkeits- oder Gewebeprobe eines Menschen und
- Vergleichen der gemessenen Menge von ApoA-IV mit einem Referenzwert.

2. Verfahren gemäß Anspruch 1, **dadurch gekennzeichnet, dass** dieser Referenzwert von einem Menschen erhalten worden ist, der keine Nierenschäden hat.

3. Verfahren gemäß Anspruch 1 oder 2, **dadurch gekennzeichnet, dass** das Frühstadium einer Nierenschädigung an Hand eines erhöhten Wertes von ApoA-IV in der Körperflüssigkeits- oder Gewebeprobe diagnostiziert wird.

4. Verfahren gemäß Anspruch 3, **dadurch gekennzeichnet, dass** dieser erhöhte Wert mindestens 20%, vorzugsweise mindestens 50%, insbesondere mindestens 70% höher ist als der Referenzwert.

5. Verfahren gemäß Anspruch 3 oder 4, **dadurch gekennzeichnet, dass** dieser Referenzwert zwischen 10 und 18 mg ApoA-IV/dl Serum beträgt.

6. Verfahren gemäß einem der Ansprüche 3 bis 5, **dadurch gekennzeichnet, dass** ein Frühstadium einer Nierenschädigung an Hand eines Wertes von höher als 18, vorzugsweise höher als 22, insbesondere höher als 28 mg ApoA-IV/dl Serum diagnostiziert wird.

7. Verfahren gemäß einem der Ansprüche 1 bis 6, **dadurch gekennzeichnet, dass** es sich bei diesem Frühstadium einer Nierenschädigung um Glomerulonephritis im Frühstadium, polyzystische Nierenerkrankung im Frühstadium, chronische "Pyelonephritis" im Frühstadium oder diabetische Nephropathie im Frühstadium handelt.

8. Verfahren gemäß einem der Ansprüche 1 bis 7, **dadurch gekennzeichnet, dass** für Menschen mit Erkrankungen der Koronararterien oder Menschen mit atherosklerotischen Komplikationen ein Referenzwert, der das Fehlen einer Nierenschädigung anzeigt, vorgesehen ist, der niedriger ist als für Menschen, die keine Nierenschädigung und keine Erkrankungen der Koronararterie oder atherosklerotische Komplikationen aufweisen.

9. Verfahren gemäß Anspruch 8, **dadurch gekennzeichnet, dass** dieser niedrigere Referenzwert, der das Fehlen einer Nierenschädigung im Frühstadium anzeigt, mindestens 20%, vorzugsweise mindestens 40%, insbesondere mindestens 60% niedriger ist als der Referenzwert für Menschen, die keine Nierenschädigung und auch keine Erkrankungen der Koronararterie oder atherosklerotische Komplikationen haben.

10. Verfahren gemäß Anspruch 8 oder 9, **dadurch gekennzeichnet, dass** dieser niedrigere Referenzwert zwischen 6 und 14 mg ApoA-IV/dl Serum beträgt.

11. Verfahren gemäß einem der Ansprüche 8 bis 10, **dadurch gekennzeichnet, dass** ein Frühstadium einer Nierenschädigung dieses Menschen mit einer Erkrankung der Koronararterie oder mit atherosklerotischen Komplikationen an Hand eines Wertes von höher als 14, vorzugsweise höher als 18, insbesondere höher als 22 mg ApoA-IV/dl Serum diagnostiziert wird.

12. Verwendung eines Verfahrens gemäß einem der Ansprüche 1 bis 11 zur Beobachtung der Entwicklung einer Nierenschädigung im Frühstadium.

13. Verwendung gemäß Anspruch 12, worin es sich bei dieser Nierenschädigung um Glomerulonephritis, eine polyzystische Nierenerkrankung, chronische "Pyelonephritis" oder diabetische Nephropathie im Frühstadium handelt.

14. Verwendung gemäß Anspruch 12 zur Differentialdiagnose von Menschen mit Erkrankungen der Koronararterien oder Menschen mit atherosklerotischen Komplikationen.

15. Verwendung eines Kits, umfassend:
- ein Gefäß mit einer Probe von Körperflüssigkeit oder einer Gewebeprobe eines Menschen mit Nierenschädigung oder dem Risiko einer Nierenschädigung, oder ein Gefäß, von dem angenommen wird, dass es mit dieser Probe gefüllt ist,
- Mittel zum Messen der Menge von ApoA-IV in einer Körperflüssigkeits- oder Gewebeprobe und
- ein Referenzwertmittel zur Ermöglichung der Diagnose einer Nierenschädigung im Frühstadium bei diesem Menschen,
zur Durchführung des Verfahrens gemäß einem der Ansprüche 1 bis 11.

16. Verwendung gemäß Anspruch 15, **dadurch gekennzeichnet, dass** das Mittel zum Messen der Menge von ApoA-IV aus Anti-ApoA-IV-Antikörpern, insbesondere polyklonalen Antikörpern, sekundären Antikörpern, insbesondere enzymatisch oder chemisch markierten sekundären Antikörpern, ApoA-IV-RNA-spezifischen Nukleinsäuren, ApoA-IV- spezifischen enzymatischen Tests, ApoA-IV-spezifischen ELISAs oder Kombinationen davon ausgewählt ist.

17. Verwendung gemäß Anspruch 15 oder 16, **dadurch gekennzeichnet, dass** dieser Referenzwert aus eine Gefäß mit einer vorbestimmten Menge ApoA-IV, einem Gefäß mit einer Körperflüssigkeits- oder Gewebeprobe eines gesunden Menschen, einem Gefäß mit einer Körperflüssigkeits- oder Gewebeprobe eines Menschen mit Nierenschädigung, jeweils vorzugsweise in lyophilisierter Form, einer Kalibrierungskurve, einer Anleitung zur Verwendung des Kits oder Kombinationen davon ausgewählt ist.

18. Verfahren zur Diagnose von koronaren Herzerkrankungen bei Menschen, die an einer Nierenschädigung im Frühstadium leiden, **gekennzeichnet durch** die folgenden Schritte:
- Messen der Menge von ApoA-IV in einer Körperflüssigkeits- oder Gewebeprobe eines Menschens und
- Vergleichen der gemessenen Menge ApoA-IV mit einem Referenzwert.

## Claims

1. A method of diagnosing early stage renal impairment in humans, **characterized by** the following steps:
- measuring the amount of apoA-IV in a body fluid or tissue sample of a human, and
- comparing the measured amount of apoA-IV with a reference value.

2. A method according to claim 1, **characterized in that** this reference value is obtained from a human without renal impairment.

3. A method according to claim 1 or 2, **characterized in that** the early stage of a renal impairment is diagnosed by way of an increased level of apoA-IV in the body fluid or tissue sample.

4. A method according to claim 3, **characterized in that** this increased value is higher by at least 20%, preferably by at least 50%, in particular by at least 70% than the reference value.

5. A method according to claim 3 or 4, **characterized in that** this reference value ranges between 10 and 18 mg of apoA-IV/dl of serum.

6. A method according to any one of claims 3 to 5, **characterized in that** an early stage of a renal impairment is diagnosed by way of a value of higher than 18, preferably higher than 22, in particular higher than 28 mg of apoA-IV/dl of serum.

7. A method according to any one of claims 1 to 6, **characterized in that** this early stage renal impairment is early stage glomerulonephritis, early stage polycystic kidney disease, early stage chronic "pyelonephritis", or early stage diabetic nephropathy.

8. A method according to any one of claims 1 to 7, **characterized in that** for humans with coronary artery diseases or for humans with atherosclerotic complications, a reference value indicating the absence of renal impairment is provided which is lower than for humans without a renal impairment and without coronary artery diseases or atherosclerotic complications.

9. A method according to claim 8, **characterized in that** this lower reference value which indicates the absence of an early stage renal impairment is lower by at least 20%, preferably by at least 40%, in particular by at least 60% than the reference value for humans without renal impairment and without coronary artery diseases or atherosclerotic complications.

10. A method according to claim 8 or 9, **characterized in that** this lower reference value is between 6 and 14 mg of apoA-IV/dl of serum.

11. A method according to any one of claims 8 to 10, **characterized in that** an early stage renal impairment of this human with a coronary artery disease or with atherosclerotic complications is diagnosed by way of a level of higher than 14, preferably higher than 18, in particular higher than 22 mg of apoA-IV/dl of serum.

12. Use of a method according to any one of claims 1 to 11 for observing the development of an early stage renal impairment.

13. Use according to claim 12, wherein this renal impairment is an early stage glomerulonephritis, polycystic kidney disease, chronic "pyelonephritis" or diabetic nephropathy.

14. Use according to claim 12 for the differential diagnosis of humans with coronary artery diseases or of humans with atherosclerotic complications.

15. The use of a kit comprising:
- a vessel containing a sample of a body fluid or a tissue sample from a human suffering from renal impairment or who is at risk of renal impairment, or a vessel assumed to be filled with this sample,
- means for measuring the amount of apoA-IV in a body fluid or tissue sample, and
- a reference value means for enabling the diagnosis of early stage renal impairment in this human,
for carrying out the method according to any one of claims 1 to 11.

16. Use according to claim 15, **characterized in that** this means for measuring the amount of apoA-IV is selected from anti-apoA-IV antibodies, in particular polyclonal antibodies, secondary antibodies, in particular enzymatically or chemically labeled secondary antibodies, apoA-IV-RNA-specific nucleic acids, apoA-IV-specific enzymatic tests, apoA-IV-specific ELISAs or combinations thereof.

17. Use according to claim 15 or 16, **characterized in that** this reference value is selected from a vessel containing a predetermined amount of apoA-IV, a vessel containing a body fluid or tissue sample of a healthy human, a vessel containing a body fluid or tissue sample of a human with renal impairment, each preferably in lyophilized form, a calibration curve, instructions for using the kit, or combinations thereof.

18. A method of diagnosing coronary heart disease in humans who suffer from (early stage) renal impairment, **characterized by** the following steps:
- measuring the amount of apoA-IV in a body fluid or tissue sample of a human, and
- comparing the measured amount of apoA-IV with a reference value.

## Revendications

1. Procédé de diagnostic de lésions rénales au stade précoce chez un être humain, **caractérisé par** les étapes suivantes :
- mesure de la quantité d'ApoA-IV dans un échantillon de fluide corporel ou un échantillon de tissu d'un être humain et
- comparaison de la quantité mesurée d'ApoA-IV à une valeur de référence.

2. Procédé selon la revendication 1, **caractérisé en ce que** cette valeur de référence a été obtenue d'un être humain qui ne présente pas de lésion rénale.

3. Procédé selon la revendication 1 ou 2, **caractérisé en ce que** le stade précoce d'une lésion rénale est diagnostiqué sur la base d'une augmentation de la valeur de l'ApoA-IV dans l'échantillon de fluide corporel ou de tissu.

4. Procédé selon la revendication 3, **caractérisé en ce que** cette valeur augmentée est d'au moins 20 %, de préférence d'au moins 50 %, en particulier, d'au moins 70 % supérieure à la valeur de référence.

5. Procédé selon la revendication 3 ou 4, **caractérisé en ce que** cette valeur de référence se situe entre 10 et 18 mg d'ApoA-IV/dl de sérum.

6. Procédé selon l'une des revendications 3 à 5, **caractérisé en ce qu'**un stade précoce d'une lésion rénale est diagnostiqué sur la base d'une valeur supérieure à 18, de préférence supérieure à 22, en particulier, supérieure à 28 mg d'ApoA-IV/dl de sérum.

7. Procédé selon l'une des revendications 1 à 6, **caractérisé en ce qu'**à ce stade précoce d'une lésion rénale, il s'agit d'une glomérulonéphrite de stade précoce, une maladie rénale polykystique de stade précoce, une "pyélonéphrite" chronique de stade précoce ou une néphropathie diabétique de stade précoce.

8. Procédé selon l'une des revendications 1 à 7, **caractérisé en ce qu'**il est prévu pour des êtres humains souffrant de maladies des artères coronaires ou pour des êtres humains souffrant de complications athéroscléreuses, une valeur de référence, qui indique l'absence d'une lésion rénale, qui est inférieure à celle des êtres humains, qui ne présentent pas de lésion rénale ni de maladies des artères coronaires ou de complications athéroscléreuses.

9. Procédé selon la revendication 8, **caractérisé en ce que** cette valeur de référence inférieure qui indique l'absence d'une lésion rénale au stade précoce est d'au moins 20 %, de préférence d'au moins 40 %, en particulier d'au moins 60 % inférieure à la valeur de référence pour les êtres humains qui ne présentent pas de lésion rénale ni de maladies des artères coronaires ou de complications athéroscléreuses.

10. Procédé selon la revendication 8 ou 9, **caractérisé en ce que** cette valeur de référence inférieure se situe entre 6 et 14 mg d'ApoA-IV/dl de sérum.

11. Procédé selon l'une des revendications 8 à 10, **caractérisé en ce qu'**un stade précoce d'une lésion rénale de ces êtres humains souffrant d'une maladie des artères coronaires ou de complications athéroscléreuses est diagnostiqué sur la base d'une valeur supérieure à 14, de préférence supérieure à 18, en particulier supérieure à 22 mg d'ApoA-IV/dl de sérum.

12. Utilisation d'un procédé selon l'une des revendications 1 à 11, pour l'observation du développement d'une lésion rénale de stade précoce.

13. Utilisation selon la revendication 12, dans laquelle cette lésion rénale est la glomérulonéphrite, une maladie rénale polykystique, une "pyélonéphrite" chronique ou une néphropathie diabétique de stade précoce.

14. Utilisation selon la revendication 12 pour le diagnostic différentiel d'êtres humains souffrant de maladie des artères coronaires ou d'êtres humains souffrant de complications athéroscléreuses.

15. Utilisation d'un kit comprenant
- un récipient comportant un échantillon de fluide corporel ou un échantillon de tissu d'un être humain souffrant d'une lésion rénale ou présentant un risque de lésion rénale ou un récipient supposé être rempli de cet échantillon,
- un moyen de mesure de la quantité d'ApoA-IV dans un échantillon de fluide corporel ou de tissu et
- un moyen de valeur de référence pour permettre le diagnostic d'une lésion rénale de stade précoce chez cet être humain, pour la réalisation du procédé selon l'une des revendications 1 à 11.

16. Utilisation selon la revendication 15, **caractérisée en ce que** l'agent de mesure de la quantité d'ApoA-IV est choisi parmi les anticorps anti-ApoA-IV, en particulier, les anticorps polyclonaux, les anticorps secondaires, en particulier les anticorps enzymatiques ou secondaires marqués chimiquement, les acides nucléiques spécifiques de l'ARN de l'ApoA-IV, les tests enzymatiques spécifiques de l'ApoA-IV, les tests ELISA spécifiques de l'ApoA-IV ou des combinaisons de ceux-ci.

17. Utilisation selon la revendication 15 ou 16, **caractérisée en ce que** cette valeur de référence est choisie parmi un récipient comportant une quantité prédéfinie d'ApoA-IV, un récipient comportant un échantillon de fluide corporel ou de tissu d'un être humain sain, un récipient comportant un échantillon de fluide corporel ou de tissu d'un être humain souffrant de lésion rénale, respectivement, de préférence sous forme lyophilisées, une courbe d'étalonnage, un mode d'emploi pour l'utilisation du kit ou une combinaisons de ceux-ci.

18. Procédé de diagnostic de coronaropathies chez l'être humain, qui souffre d'une lésion rénale de stade précoce, **caractérisé par** les étapes suivantes :
- mesure de la quantité d'ApoA-IV dans un échantillon de fluide corporel ou de tissu d'un être humain et
- comparaison de la quantité mesurée d'ApoA-IV à une valeur de référence.
